# EUROPEAN PATENT APPLICATION

(11) **EP 4 523 614 A1**
(43) Date of publication of application: **19.03.2025**
(21) Application number: 23197546.7
(22) Date of filing: 14.09.2023
(51) Int. Cl.: A61B 5/021, A61B 5/024, A61B 5/0507, A61B 5/103, A61B 5/00, A61B 5/022

(54) **MULTI-SENSOR DEVICE**

(71) Applicant: Withings, 92130 Issy-Les-Moulineaux (FR)
(72) Inventor: Matthès, Maxime, 92130 Issy-les-Moulineaux (FR); Vion-Bailly, Jérémy, 92130 Issy-les-Moulineaux (FR)
(74) Representative: Withings IP

(57) **Abstract**

The invention relates to a new physiological sensor capable of using several synchronized physiological signals to identify physiological properties of a user: at least two of a load sensor, an optical sensor and a radiofrequency resonant sensor are used together to obtain data from a MUT on a interface area.

## Description

### Field of the invention

The present invention relates to physiological sensors and in particular to physiological sensor devices integrating a plurality of physiological sensors (referred to as "multisensor device").

### Background

There exist many physiological signals which can be measured with physiological sensors. Some of them, such as heart rate, body temperature, blood pulse, respiration, etc. are well known and widely monitored using various technologies of physiological sensors.

Multi-sensor approaches have been and are explored, to monitor perturbations that can shift and alter the measurement of interest: variations of temperature, humidity, blood perfusion, tissue compression, movement, etc. The following papers provide some pieces of existing technologies.

Turgul et al. (V. Turgul and I. Kale, "A novel pressure sensing circuit for non-invasive RF/microwave blood glucose sensors," 2016 16th Mediterranean Microwave Symposium (MMS), Abu Dhabi, United Arab Emirates, 2016, pp. 1-4, doi: 10.1109/MMS.2016.7803818) discloses a radiofrequency (RF) sensor coupled with a load sensor, aimed at mitigating the influence of exerted pressure on the RF signal (see IV. B for example). One of the outcomes, set out in Fig. 10 is the almost linear response between resonance frequency and pressure.

Caduff et al. (Caduff A, Mueller M, Megej A, Dewarrat F, Suri RE, Klisic J, Donath M, Zakharov P, Schaub D, Stahel WA, Talary MS. Characteristics of a multisensor system for non invasive glucose monitoring with external validation and prospective evaluation. Biosens Bioelectron. 2011 May 15;26(9):3794-800. doi: 10.1016/j.bios.2011.02.034. Epub 2011 Apr 13. PMID: 21493056.)" disclosed a sensor with MHz electrode and optical sensors LEDs (green, red and infrared).

Omer et al (Omer, A.E., Shaker, G., Safavi-Naeini, S. et al. Low-cost portable microwave sensor for non-invasive monitoring of blood glucose level: novel design utilizing a four-cell CSRR hexagonal configuration. Sci Rep 10, 15200 (2020). https://doi.org/10.1038/s41598-020-72114-3) discloses a Complementary Split Ring Resonator architecture.

Combining signals from different sensors provides further insight about physiological data of a user but there is much left to be done.

### Summary of the disclosure

An aim of the disclosure is therefore to provide a new physiological sensor capable of using several synchronized physiological signals to identify physiological properties of a user.

The invention is defined in the appended claims.

In an embodiment, the disclosure relates to a physiological sensor device incorporating a plurality of sensors cooperating together to generate physiological data. In particular, one of the sensors is a radiofrequency sensor, whose transmission or reflection frequency spectrum, which is measurable, depends on the material under test, MUT. The MUT is a tissue zone, such as a finger or more precisely such as a fingertip.

To that end, an aspect of the disclosure relates to a physiological sensor device comprising:
- a resonant radiofrequency (RRF) sensor comprising an antenna, the RRF sensor being configured to generate resonance data,
- a optical sensor, configured to generate optical data,
- a load sensor, configured to generate load data,
- control circuitry,

wherein the physiological sensor device includes an interface area configured to receive a part of a user body, such as a finger,
wherein the antenna and the optical sensor are arranged at the interface area, and the load sensor is configured to measure a load exerted on the interface area

An aspect of the disclosure relates to a physiological sensor system comprising the physiological sensor device and control circuitry, wherein control circuitry is configured to:
- determine resonance data using the resonance radiofrequency sensor, optical data using the optical sensor, load data using the load sensor, the resonance data, the optical data and the load data being temporally synchronized,
- determine augmented physiological data by combining the synchronized resonance data, optical data and load data.

The interface area has a small surface, so that the same part of the user body is simultaneously measured by the three sensors.

When performing a PPG measurement, the finger exerts a force on the sensor. The reaction force, exerted by the sensor on the finger, compresses tissues and affects the backscattered PPG signal. However, tissues are not deformed in the same fashion when the contact area varies for a given force. Instead of a force measurement, a pressure measurement would be more appropriate. Using the load sensor and the RRF sensor, more knowledge about the MUT may be obtained to improve optical data or to better analyze the optical data.

In an implementation, the resonant radiofrequency sensor includes a split-ring resonator (SRR) or a complementary split-ring resonator (CSSR).

In an implementation, the load sensor includes a load support configured to receive a load exerted on the interface area.

In an implementation, the optical sensor is mounted on the load support.

In an implementation, the RFF sensor comprises a substrate on which the antenna is mounted and the substrate has an aperture for the optical sensor.

In an implementation the resonant radiofrequency sensor is configured to work between 1 GHz and 10 GHz.

In an implementation the interface area is less than 2 cm², even less than 1cm².

In an implementation the sensor device comprises a non-conducting dielectric layer covering the RFF sensor. The interface area may be located on the non-conducting dielectric layer.

In an implementation, determining augmented physiological data comprises:
- determining pressure data of a pressure exerted on the finger MUT using the load data and the resonance data; and
- combining optical data and pressure.

In an implementation, determining augmented physiological data further comprises:
- determining cleansed optical data using the combined optical data and pressure data.

In an implementation determining augmented physiological data further comprises:
- determining an oscillometric measurement of the blood pressure of the user using the combined optical data and pressure data.

In an implementation, the optical data includes an envelope of the optical data and determining an oscillometric measurement includes analysing the envelope using the pressure data.

Another aspect of the disclosure relates to a method using the system described previously.

Another aspect of the disclosure relates to a physiological sensor system comprising a physiological sensor device and control circuitry, the physiological sensor device comprising:
- a resonant radiofrequency (RRF) sensor comprising an antenna, configured to generate resonance data,
- a optical sensor, configured to generate optical data,
- control circuitry,

wherein the physiological sensor device includes an interface area configured to receive a part of a user body, such as a finger,
wherein the antenna and the optical sensor are arranged at the interface area,
wherein control circuitry is configured to:

- determine resonance data using the resonance radiofrequency sensor and optical data using the optical sensor, load data using the load sensor, the resonance data, the optical data and the load data being temporally synchronized,
- determine augmented physiological data by combining the synchronized resonance data and optical data.

The disclosure presents a multi-sensor device which gathers information originated from the same tissue zone. This allows for the improvement of the signal processing of a PPG signal or a resonant signal, thanks to the other signal. In other words, the multiplicity of sensors permits the disambiguation of the origin of signals from tissues alteration.

In an implementation, the resonant radiofrequency sensor includes a split-ring resonator (SRR) or a complementary split-ring resonator.

In an implementation, the RFF sensor comprises a substrate on which the antenna is mounted and the substrate has an aperture for the optical sensor.

In an implementation, the resonant radiofrequency sensor is configured to work between 1 GHz and 10 GHz.

In an implementation, the interface area is less than 2 cm², even less than 1 cm².

In an implementation the sensor device comprises a non-conducting dielectric layer covering the RFF sensor. The interface area may be located on the non-conducting dielectric layer.

In an implementation determining augmented physiological data comprises:
- determining, for a varying load of the MUT on the interface area, resonance data,
- determining optical data when resonance data is above a predetermined threshold.

In an implementation, determining augmented physiological data comprises:
- determining, for a varying load of the MUT on the interface area, optical data,
- determining resonance data when optical data is above a predetermined threshold.

In an implementation, optical data comprises a pulsative component of a pulsative optical signal.

Another aspect of the disclosure relates to a method using the system described previously.

### Brief description of the Drawings

These features and advantages of the invention will appear more clearly upon reading the following description, provided solely as a non-limiting example, and done in reference to the appended drawings, in which:
Figure 1 shows two sides of a device according to an embodiment of the disclosure,
Figure 2 shows the device of Figure 1 with control circuitry,
Figure 3 shows a user's fingertip on the device according to another embodiment of the disclosure,
Figure 4 shows a method according to an embodiment, using the RRF sensor, the load sensor and the optical sensor,
Figure 5 shows two implementations of the method of Figure 4,
Figure 6 shows a method according to another embodiment, using the RRF sensor and the optical sensor,
Figure 7 shows data obtained using the RRF sensor and the optical sensor, with a varying load,
Figure 8 shows an implementation of the method of Figure 6, and
Figure 9 shows another implementation of the method of Figure 6.

### Detailed description

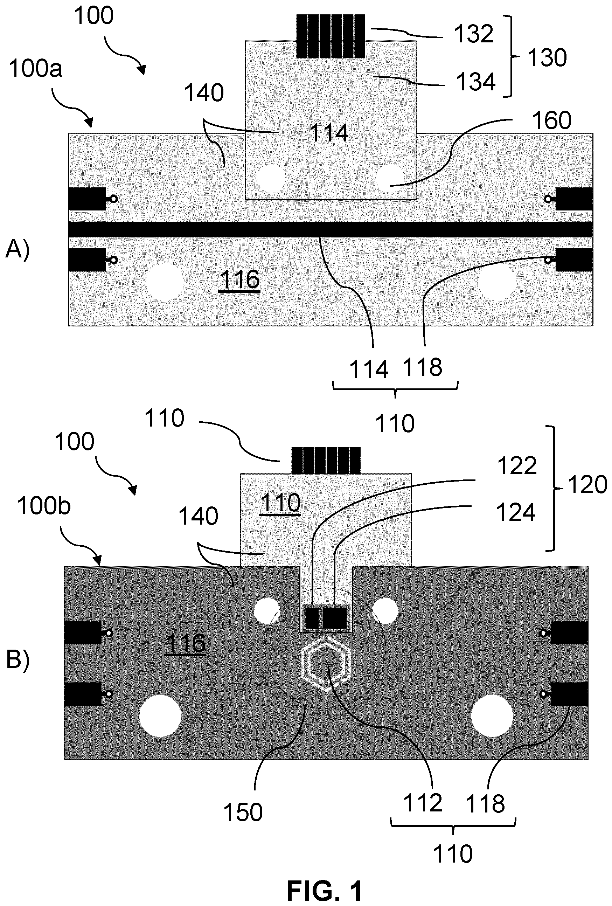

Figure 1 illustrates an embodiment of a physiological sensor device 100, referred to as device 100. Device 100 is configured to generate signals which depend on a material under test ("MUT"). The MUT is here a part of a user's body, in particular a tissue zone, such as a finger or a fingertip. Device 100 may include a back side 100a (shown in Figure 1 A) and a front side 100b (shown in Figure 1 B).

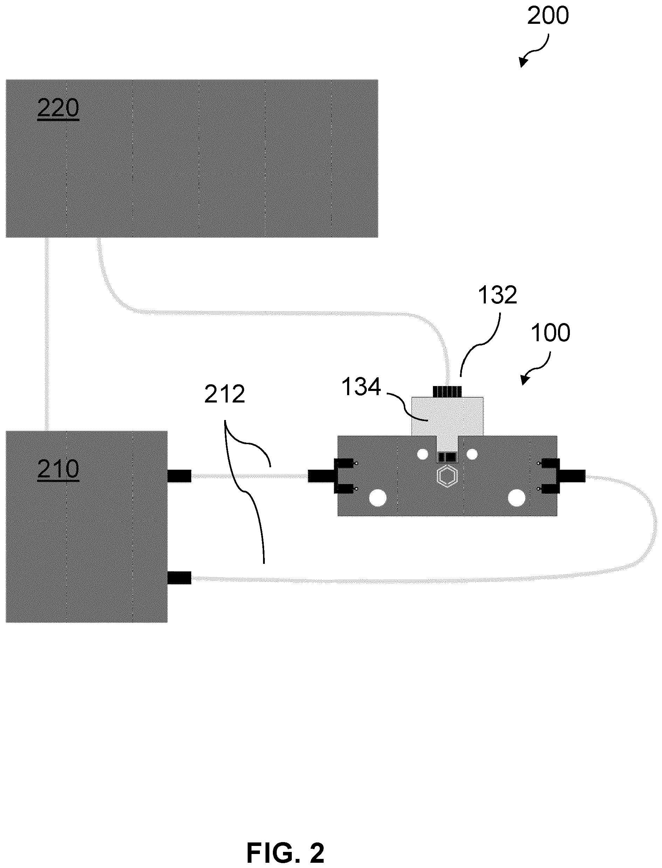

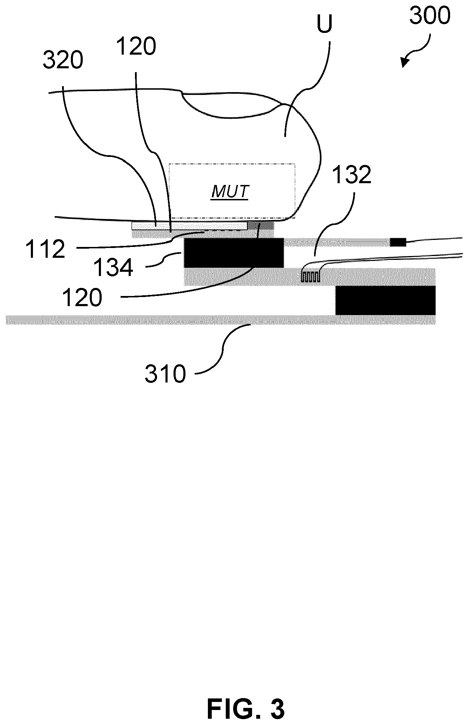

Figure 2 illustrates device 100 as part of a physiological sensor system 200, referred to as system 200. Figure 3 illustrates a fingertip of a user U and another embodiment of the device 100 as incorporated in a (portion of) system 200, with a portion as mounted on an apparatus 300 for use at home (point-of-care), such as a handheld device (nor represented). Only a support 310 of apparatus 300 is visible. The dashed rectangle represents schematically the MUT, that is to say the material whose properties affect the RFF sensor 110 and the optical sensor 120.

Device 100 comprises at least two sensors from three sensors 110, 120, 130 mounted on a support assembly 140. The plurality of sensors includes at least two sensors, or more particularly at least three sensors. Sensor 110 is a resonant radiofrequency sensor, referred to as RRF sensor 110 in the rest of the description, and configured to generate resonant radiofrequency data ("RRF data"). Sensor 120 is an optical sensor, such as a photoplethysmogram (PPG) sensor, configured to generate optical data. Sensor 130 is a load sensor, configured to generate load data. More particularly, device 100 is configured to generate those three types of data in a synchronized manner. This involves also a parallel acquisition of signal on the MUT using the sensors 110, 120, 130.

The at least two sensors 110, 120, 130 are arranged close to one another so that they measure physiological properties of a same MUT (e.g., a portion of a finger). To that end, device 100 includes an interface area 150, which corresponds to an area on which the MUT must be placed. The interface area is small enough so that the same part of the MUT is measured by the sensors. The interface area 150 may be sized so that it is entirely covered by the MUT.

For example, the interface area 150 may be a recess in a case (e.g., plastic case) of the apparatus 300 so that the user knows where and how to put the MUT (the fingertip essentially). Alternatively or complementarily, the interface area 150 may be of a specific color or surface, for a similar purpose.

The interface area 150 has to be small enough so that measurements by at least two of the three sensors 110, 120, 103 are carried out for a same tissue zone; otherwise, the very purpose of the multi-sensor approach of the present disclosure becomes moot.

As illustrated on Figure 3, the interface area 150 may be located on a non-conducting dielectric layer 320 (glass or plastic) to protect the RRF sensor 110 without interfering or with a known interference with the RRF sensor 110, and to prevent the short-circuit of the RRF sensor 110.

For example, the interface area 150 has a surface which is less than 2 cm², even less 1 cm², for example a circular surface with a diameter less than 1 cm.

System 200 comprises control circuitry 220 which typically includes a processor, a memory and an I/O interface to receive and send data.

### Resonant radiofrequency sensor 110

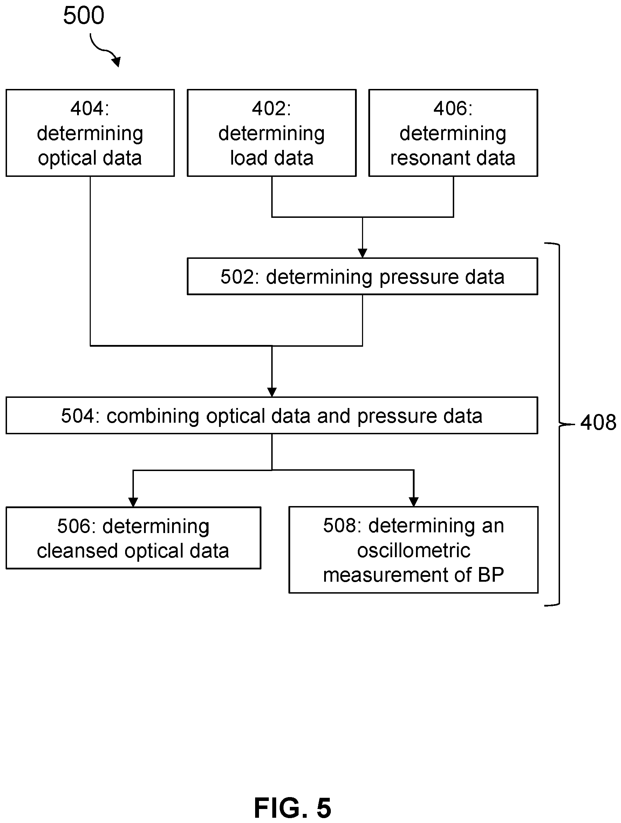

The RFF sensor 110 is able to generate RFF signals treated by control circuitry 220 to determine resonance data. Example of resonance data may be found in Figure 5 of in Ansari et al. (M. A. H. Ansari, A. K. Jha and M. J. Akhtar, "Design and Application of the CSRR-Based Planar Sensor for Noninvasive Measurement of Complex Permittivity," in IEEE Sensors Journal, vol. 15, no. 12, pp. 7181-7189, Dec. 2015, doi: 10.1109/JSEN.2015.2469683). The antenna 112 may have different shapes as it has been and is being explored (see Abdolrazzaghi et al.: Abdolrazzaghi, M.; Nayyeri, V.; Martin, F. Techniques to Improve the Performance of Planar Microwave Sensors: A review and Recent Developments. Sensors 2022, 22, 6946. https://doi.org/10.3390/ s22186946).

As visible on the top side 100b, sensor 110 may include an antenna 112, also called resonant structure. The MUT changes resonance frequencies of the antenna 112, notably because of the dielectric properties of the MUT. The antenna 112 receives an input signal Sin in the form of an electromagnetic radiation in the radiofrequency spectral domain through a transmission line 114 (often called "microstrip"), which may be located on the back side 100a. In response the antenna generates an electromagnetic fields, whose properties depends on the MUT, notably the dielectric properties of the MUT. It has been shown that those dielectric properties depend on the nature of the tissue (blood, skin, interstitial liquid, etc.) and the state of the tissue (free, compressed). See for instance S Gabriel et al 1996 Phys. Med. Biol. 41 2251 (DOI 10.1088/0031-9155/41/11/002), "The dielectric properties of biological tissues: II. Measurements in the frequency range 10 Hz to 20 GHz". The input signal IS is generated by a RF control unit 210, which is itself controlled by control circuitry 220. Control unit 210 includes a voltage generator capable of sending notably RF signals of sweeping frequency ("chirps"). The transmission line 114 and the RF control unit 210 are connected through electrical links 212 (such as coaxial cable, such as SMA, and/or waveguides). The antenna 112 is excited by the input IS and its resonance frequencies are measured by the RF module 220 by means of the output signal S*out.*

The antenna 112 is typically located in the interface area 150. The antenna 112 may be mounted on a substrate 116, which is attached at least indirectly to the support 310 (although not illustrated on Figure 3). The support 310 is part of the structure of sensor system 300 or may be an intermediate piece attached to the structure of the sensor system. In an embodiment, the substrate 116 is made of plastic.

Connection pins 118 may be provided to connect the transmission line 114 (e.g., coaxial cable) to the ground of the resonance sensor 110 (the ground being the dark grey part of substrate 116 on Figure 1 B).

Using the "s-parameter" common terminology for RRF sensors, RF control unit 210 is configured to compute S11 and S12 from measured signals for example. To that end, the RF control unit 210 may for example include a voltage controlled oscillator VCO and an amplitude detector, or a VCO and a spectral analyzer, or a vector network analyzer VNA.

Sensor 110 is advantageously planar for a better mechanical integration in the sensor device and for an optical geometrical compatibility with the MUT.

Sensor 110 may be built by etching technology, by removing conductive matter on substrate 116.

For example, a type of sensor 110 fulfilling all the requirements mentioned above is a complementary split ring resonator (CSSR). This technology has been described in many papers such as those mentioned in the introduction, such as in Ansari et al. (M. A. H. Ansari, A. K. Jha and M. J. Akhtar, "Design and Application of the CSRR-Based Planar Sensor for Noninvasive Measurement of Complex Permittivity," in IEEE Sensors Journal, vol. 15, no. 12, pp. 7181-7189, Dec. 2015, doi: 10.1109/JSEN.2015.2469683). The antenna 112 may have different shapes as it has been and is being explored (see Abdolrazzaghi et al.: Abdolrazzaghi, M.; Nayyeri, V.; Martin, F. *Techniques to Improve the Performance of Planar Microwave Sensors: A review and Recent Developments.*

Sensors 2022, 22, 6946. https://doi.org/10.3390/ s22186946). Figure 5 of Ansari shows typical resonant data measured for a given CSRR geometry and different MUTs of different dielectric properties.

### Optical sensor 120

The optical sensor 120 is able to generate, detect and digitize optical signals treated by control circuitry 220 to determine optical data.

The optical sensor 120 includes a light emitting assembly 122 and a light receiving assembly 124, visible on top side 150. The light emitting assembly 122 is configured to send light into the MUT and the light receiving assembly is configured to receive light that went through the MUT. An example of optical sensor 120 may be found in Caduff et al. - 2011 (mentioned above), with an exemplified integration into a multi-sensor device.

The light emitting assembly 122 may include a plurality of light emitters and the light receiving assembly 124 may receive a plurality of light receivers. A path between one light emitter and one receiver is called a channel. The optical sensor 120 may therefore have a plurality of channels.

For example, the light emitter may comprise one or more LEDs and the light receiver may comprise one or more photodiodes. The optical sensor 120 may work in different wavelengths, such as green, red or infrared (or near infrared). In a variant, the light emitter may comprise one or more laser diodes.

The optical sensor 120 is arranged at the interface area 150, that is to say near the antenna 112. By near, it is meant that the RFF sensor and the optical sensor 120 measure physiological parameters of the same MUT.

In an embodiment, the optical sensor 120 is mounted on the substrate 116. For example, the optical component of the light emitting assembly 122 and the light receiving assembly 124 are soldered on substrate 116. Caduff et al. shows in Figure 1 an example of integration of an optical sensor 120 near electrodes.

To physically accommodate the optical sensor 120 in the interface area 150, the substrate 140 may include an opening (extending to an edge on Figure 1).

### Load sensor 130

Load sensor 130 is configured to measure a load applied by the MUT in the interface area 150. As visible on Figure 1, in an embodiment, load sensor 130 includes at least one load cell 132, whose measurable deformation can be linked to received load. Load sensor 130 may further comprise a load support 134, located between the MUT and the load cell(s) 132 along the load path. At least a portion of the load support 134 is in the interface area 150. The load support 134 may therefore include a portion of the resonance sensor 110 and/or the optical sensor 120.

The load sensor 130 is able to generate load signals treated by control circuitry 220 to determine load data.

The load cell(s) 132 is/are typically connected to control circuitry 220 for further treatment (such as the conversion of the electrical tension into a load). A microcontroller may be connected on the lad cells (for example on the load support 134).

When the user puts the MUT (e.g., the fingertip) on the load sensor 130, the MUT exerts a load on the load support 134, which in turn exerts a load on the load cell 132. The load plate transfers the load to the load cells 132.

As illustrated on Figures 1 to 3, load support 134 is a separate element from substrate 116 but is attached thereto, via connectors 160, so that substrate 116 and load support 134 cannot move relatively to one another. In a variant, load support 134 is integral with substrate 116.

In the embodiment where the load support 134 and the substrate 116 are fixed to one another, the load cell 132 may be placed at different locations on the device 100. For example, on figure 3, the load cell 132 is underneath (on the drawings) the optical sensor 120 and the resonance sensor 110.

In another embodiment, the load support 134 can move relatively to the substrate 116, so that the substrate 116 does not receive a strong load.

The load support 134 may be a load plate.

In an embodiment, the optical sensor 120 is mounted on the load support 134. More precisely, the load support 134 (e.g., a load plate) may extend underneath the substrate 116, facing the back side 100a so that the opening in the substrate 116 creates a space therein for the optical sensor 120, as visible on Figure 1 B. In this embodiment, the load support 134 may also be a printed circuit board (PCB) comprising electronic components for the optical sensor 110.

The interface area 150, which receives the MUT, includes therefore a portion of the substrate 116 with the antenna and the optical sensor 120 mounted on the load plate.

Other technologies of the load sensor 130 are possible, such as a shear sensor.

### Additional sensors

Other sensors may be added to the device, such as a temperature sensor and/or humidity sensor, which may affect RFF signals.

### Methods

The description will now focus on several methods using at least the RFF sensor 110 and at least one amongst the optical sensor 120 and the load sensor 130.

Before all, the user needs to put the MUT on the interface area 150. A detection system, involving for instance the load sensor 130 (by detecting a load) and/or the optical sensor 120 (by detecting that the optical sensor 120 is covered), may be used to trigger method 400.

### Method using three sensors

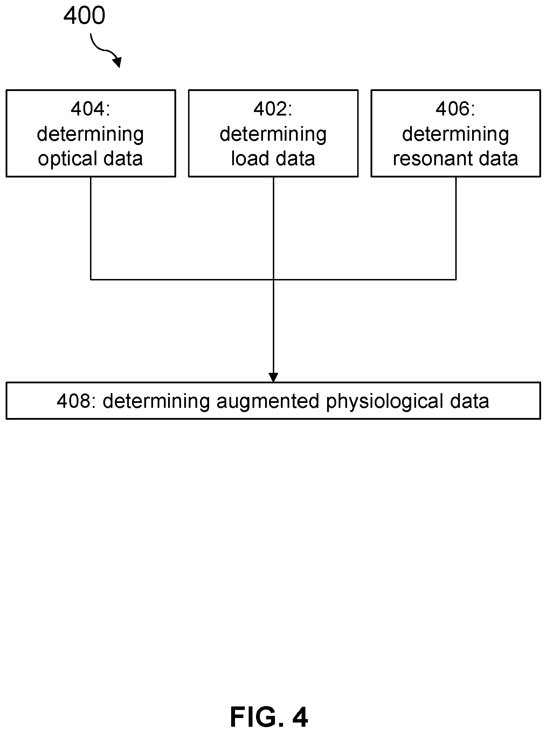

Figure 4 illustrates a generic method 400 using the three sensors 120, 130 and 140. Method 400 is carried out by control circuitry 220, using at least the sensors 120, 130 and 140.

At 402, control circuitry 220 determines optical data using one or more optical signals from the optical sensor 120.

At 404, control circuitry 220 determines load data using one or more load signals from the load sensor 130. Steps 402 and 404 are advantageously carried out in parallel.

At 406, control circuitry 220 determines resonance data using resonant signal from the RFF sensor 110. Any two or three of steps 402, 404, 405 are carried out in parallel.

Load data, optical data and resonance data may be obtained continuously during a measurement period. The measurement period may last between 10s and 60s. More specifically, load data, optical data and resonance data are synchronized (synchronized meaning acquired at the same time, i.e. during the same acquisition period and not at a same acquisition frequency: for example, the optical sensor 120 may acquire at 100 Hz while RFF sensor may acquire at 1 Hz).

At 408, control circuitry 220 determines augmented physiological data by combining the load data, the optical data and the resonance data. The augmented physiological data may for instance improve the precision of any data stemming from the perfusion index of the optical signal.

The operation frequency of the signal used at 406 may be between 1 GHz and 10 GHz. The RFF sensor 110 is designed to have its resonance frequency without the MUT at a chosen frequency.

Changing the shape of the antenna 112 may also increase the depth of resonance, the directivity of the emitted field and the penetration depth of the field in the MUT.

Figure 5 illustrates a method 500, which is a more specific embodiment of the method 400. Method 500 illustrates in more detail step 408 of determining augmented physiological data. Therefore step 408 includes the following steps.

At 502, control circuitry 220 determines pressure data exerted on the MUT, using the resonance data and the load data.

At 504, control circuitry 220 combines the optical data with the pressure data. This combination can fulfill several different objectives.

In a first embodiment, at 506, control circuitry 220 determines a cleansed optical signal by using the pressure data to clean the optical data. Indeed, optical data is highly-pressure sensitive (see for instance PCT/EP2023/059588) and the pressure data allows filtering the pressure-related effect on the optical signal. Such filtering may be carried out using machine learning algorithms. The cleansed optical data may therefore improve any measurement or data using the cleansed optical signal, in particular the perfusion index or any data stemming from the AC component of the optical signal.

In a second embodiment (which may be combined with the first embodiment), at 508 control circuitry 220 determines an oscillometric measurement of the blood pressure (BP) in the MUT using the combination of optical data and pressure data. Optical data may include here the envelope of the optical signal, such as a PPG signal and more precisely a perfusion index of the PPG signal. Pressure data helps analyzing the envelope of optical data. Perfusion indices are described in more detail in PCT/EP2023/059588). Method 400 therefore allows a user to obtain a blood pressure measurement without having to wear an inflatable cuff, as it is currently the gold standard in non-invasive methodologies.

Chowdhury et al. (Chowdhury MH, Shuzan MNI, Chowdhury MEH, Mahbub ZB, Uddin MM, Khandakar A, Reaz MBI. Estimating Blood Pressure from the Photoplethysmogram Signal and Demographic Features Using Machine Learning Techniques. Sensors (Basel). 2020 Jun 1;20(11):3127. doi: 10.3390/s20113127. PMID: 32492902; PMCID: PMC7309072) explains in more detail the relationship between optical data (PPG data here) and blood pressure.

In an embodiment, to determine resonance data at 406, control circuitry 220 sends a chirp signal via the RF control unit 210 using a vector network analyzer or a voltage controlled oscillator, coupled with an amplitude detector, to measure the transmission amplitude of signal S21. This allows to obtain the minimal resonance frequency fc. The minimal resonance frequency fc is then determined continuously during the measurement period. Alternatively, instead of signal S21, control circuitry 220 uses signal S11.

An analog radiofrequency low pass filter may be added to suppress any potential effect of the input signal harmonics on the resonance.

In an embodiment, to determine pressure data on the MUT at 502, control circuitry 220 determines a coefficient linking the resonance frequency fc with the load data. This coefficient is indicative of the pressure on the MUT.

### Method using two sensors

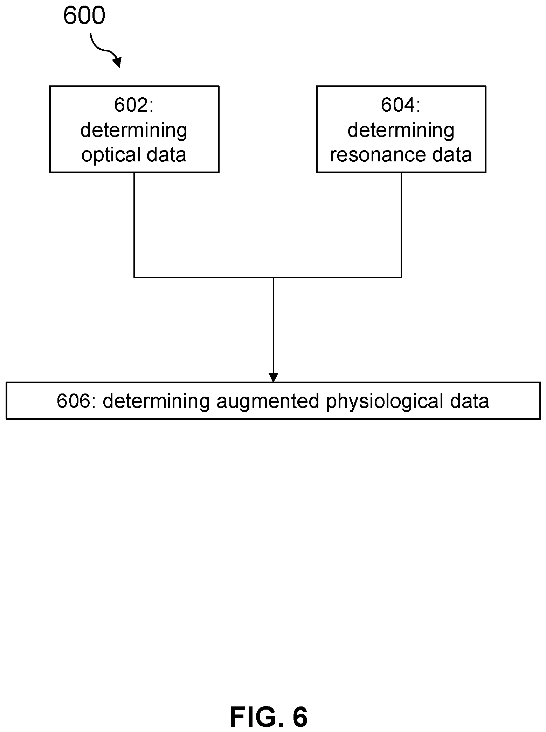

Figure 6 illustrates a method 600 using two sensors. Method 600 is carried out by control circuitry 220, using at least a pair of sensors 120, 130 and 140.

At 602, control circuitry 220 determines optical data using one or more optical signals from the optical sensor 120.

At 604, control circuitry 220 determines resonance data using resonant signal from the RFF sensor 110. Steps 602, 604 are carried out in parallel.

Optical data and resonance data may be obtained continuously during a measurement period. The measurement period may last between 10s and 60s. More specifically, the respective acquisitions optical data and resonance data are synchronized (again, synchronized meaning acquired at the same time, i.e. during the same acquisition period and not at a same acquisition frequency:).

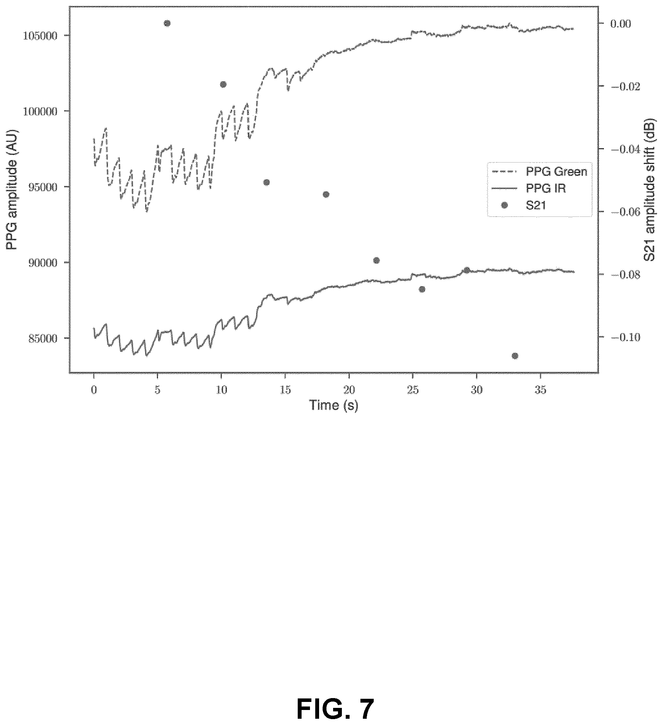

Figure 7 shows a plotted curve of raw optical data (PPG data) and resonance data (electromagnetic amplitude shift of S21 signal): dashed line is for green PPG, continuous line is for infrared PPG and the dots are for S21 signal. Curves are to be read on the left scale and dots on the right scale.

The protocol was as follows: a user exerted a pressure on the device 100, at the sensor interface 150 and the pressure was kept constant for 4 seconds (to get PPG data). As time goes, pressure increases. It can be observed that:
- the pulsatile component of the PPG data (the one which is due to the blood pulsation) tends to decrease as pressure increases,
- once blood has left the MUT, the tissues are not totally yet too compressed.

Therefore, it shows that resonance data can help to predict the quality of optical data.

Conversely, it shows that optical data can help to characterize the resonance properties of the MUT.

At 606, control circuitry 220 determines augmented physiological data by combining the optical data and the resonance data.

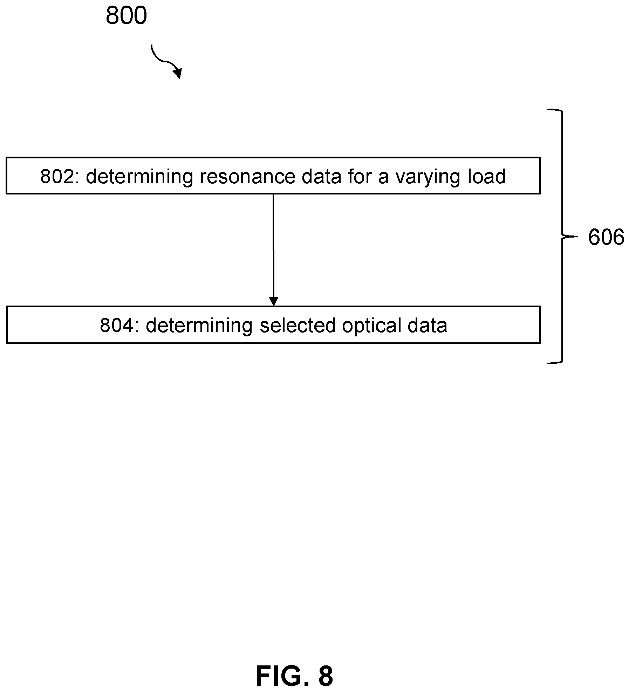

Figure 8 illustrates a method 800, which is a more specific embodiment of the method 600. Method 800 illustrates in more detail step 606 of determining augmented physiological data, wherein such data are improved optical data. This method 800 is based on the fact that resonance data provide insight about the pressure/load/local deformation of the MUT on the interface area 150.

Step 606 includes the following steps.

At 802, control circuitry 220 determines, for a varying load of the MUT on the interface area 150, resonance data. Step 802 may be included in steps 602, 604.

At 804, control circuitry 220 determines optical data when resonance data of step 802 is above or below a predetermined resonance threshold. Determining 804 may here means extracting a subset of optical data for which synchronized resonance data is above or below the predetermined resonance threshold. The predetermined resonance threshold may be obtained via prior measurement on the user or in factory. This way, using the relationship between load and/or the pressure and the resonance data, the quality of the selected subset of optical data is improved as they were selected for a load/pressure range deemed appropriate for the optical measurement (through the resonance threshold).

For example, the optical data may comprise here a pulsatile component of a pulsatile optical signal, such as the AC part of a PPG signal or a pulsatile ratio AC/DD.

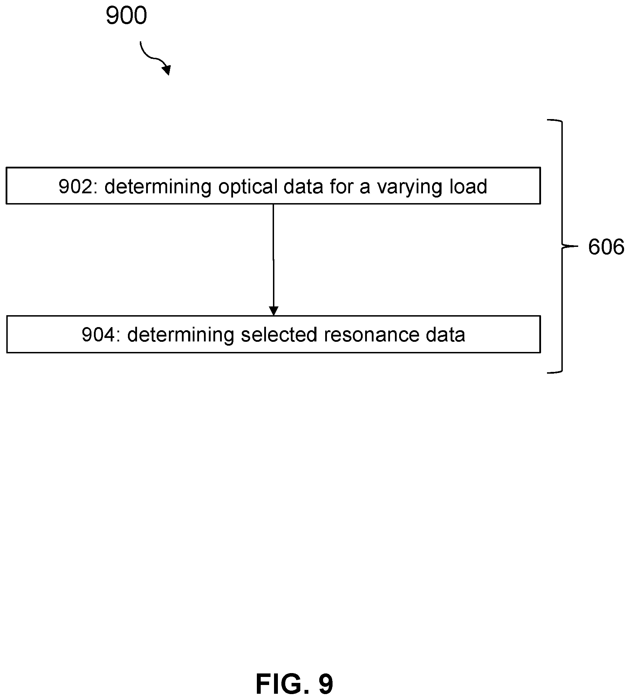

Figure 9 illustrates a method 900, which is a more specific embodiment of the method 600. Method 900 illustrates in more detail step 606 of determining augmented physiological data, wherein such data are improved resonance data. Therefore, step 608 includes the following steps.

At 902, control circuitry 220 determines, for a varying load of the MUT on the interface area 150, optical data. Steps 902 may be included in step 602, 604.

At 904, control circuitry 220 determines resonance data when optical data of step 902 is above or below a predetermined optical threshold. Determining 904 may here means extracting a subset of resonance data for which synchronized optical data is above or below the predetermined optical threshold. The predetermined optical threshold may be obtained via prior measurement on the user or in factory. This way, using the relationship between load and/or the pressure and the optical data, the quality of the selected subset of resonance data is improved as they were selected for a load/pressure range deemed appropriate for the optical measurement (through the resonance threshold).

For example, the optical data may comprise here a pulsatile component of a pulsatile optical signal, such as the AC part of a PPG signal.

For methods 800 and 900, a step of identifying a load variation may be added, to ensure that method 600 is properly carried out. Load variation may be identified through optical data (as observed before) or resonance data (as observed before).

In addition, the load sensor 130 may be included and load data may be determined by control circuitry to identify that a load is varying and/or to provide further data for the optical data and resonance data.

### Clause :

The following clauses relates to another aspect of the disclosure compared to that defined in the claims as filed :
1. A physiological sensor system (200) comprising a physiological sensor device (100) and control circuitry (210), the physiological sensor device (100) comprising:
   - a resonant radiofrequency sensor, RRF, (110) comprising an antenna (112), the resonant radiofrequency sensor (110) being configured to generate resonance data,
   - a optical sensor (120), configured to generate optical data,
   - control circuitry (210),

   wherein the physiological sensor device (100) includes an interface area (150) configured to receive a part of a user body, such as a finger,
   wherein the antenna (112) and the optical sensor (120) are arranged at the interface area (150),
   wherein control circuitry (210) is configured to:
      - determine resonance data using the resonance radiofrequency sensor (110) and optical data using the optical sensor (120), load data using the load sensor (13), the resonance data, the optical data and the load data being temporally synchronized,
      - determine augmented physiological data by combining the synchronized resonance data and optical data.
2. The physiological sensor system (200) of clause 1, wherein the resonant radiofrequency sensor includes a split-ring resonator (SRR) or a complementary split-ring resonator (CSSR 200).
3. The physiological sensor system (200) of any of clauses 1-2, further comprising a non-conducting dielectric layer (320) covering the RFF sensor (110).
4. The physiological sensor system (200) of clause 3, wherein the interface area is located on the non-conducting dielectric layer (320).
5. The physiological sensor system (200) of any of clauses 1-4, wherein the RFF sensor comprises a substrate on which the antenna is mounted and the substrate has an aperture for the optical sensor.
6. The physiological sensor system (200) of any of clauses 1-5, wherein the resonant radiofrequency sensor (110) is configured to work between 1 GHz and 10 GHz.
7. The physiological sensor system (200) of any of clauses 1-6, wherein the interface area is less than 2 cm².
8. The physiological sensor system (200) of any of clauses 1-7, wherein determining augmented physiological data comprises:
   - determining, for a varying load of the MUT on the interface area (150), resonance data,
   - determining optical data when resonance data is above a predetermined threshold.
9. The physiological sensor system (200) of any of clauses 1-8, wherein determining augmented physiological data comprises:
   - determining, for a varying load of the MUT on the interface area, optical data,
   - determining resonance data when optical data is above a predetermined threshold.
10. The physiological sensor system (200) of clauses 8 or 9, wherein optical data comprises a pulsatile component of a pulsatile optical signal.
11. A method using a physiological sensor system (200) as defined in any of clauses 1-10, wherein control circuitry (210) is configured to:
   - determine (406) resonance data using the resonance radiofrequency sensor (110), optical data (404) using the optical sensor (120), load data (402) using the load sensor (130); the resonance data, the optical data and the load data being temporally synchronized,
   - determine (408) augmented physiological data by combining the synchronized resonance data, optical data and load data.

## Claims

1. A physiological sensor system (200) comprising a physiological sensor device (100) and control circuitry (210), the physiological sensor device comprising:
- a resonant radiofrequency sensor (110) comprising an antenna (112), the resonant radiofrequency sensor (110) being configured to generate resonance data,
- a optical sensor (120) configured to generate optical data,
- a load sensor (130) configured to generate load data,
wherein the physiological sensor device (100) includes an interface area (150) configured to receive a part of a user body, such as a finger,
wherein the antenna (112) and the optical sensor (120) are arranged at the interface area (150), and the load sensor (130) is configured to measure a load exerted on the interface area (150),
wherein control circuitry (210) is configured to:
- determine (406) resonance data using the resonance radiofrequency sensor (110), optical data (404) using the optical sensor (120), load data (402) using the load sensor (130); the resonance data, the optical data and the load data being temporally synchronized,
- determine (408) augmented physiological data by combining the synchronized resonance data, optical data and load data.

2. The physiological sensor system (200) of claim 1, wherein the resonant radiofrequency sensor includes a split-ring resonator (SRR) or a complementary split-ring resonator (CSSR 200).

3. The physiological sensor system (200) of any of claims 1-2, wherein the load sensor (130) includes a load support (134) configured to receive a load exerted on the interface area (150).

4. The physiological sensor system (200) of claim 3, wherein the optical sensor (130) is mounted on the load support (134).

5. The physiological sensor system (200) of any of claims 1-4, wherein the resonant radiofrequency sensor (110) comprises a substrate (116) on which the antenna (112) is mounted and the substrate (116) has an aperture for the optical sensor (120).

6. The physiological sensor system (200) of any of claims 1-5, wherein the resonant radiofrequency sensor (110) is configured to work between 1 GHz and 10 GHz.

7. The physiological sensor system (200) of any of claims 1-6, wherein the interface area (150) is less than 2 cm².

8. The physiological sensor system (200) of any of claims 1-7, wherein determining augmented physiological data (408) comprises:
- determining pressure data (502) of a pressure exerted on the MUT using the load data and the resonance data; and
- combining optical data and pressure (504).

9. The physiological sensor system (200) of claim 8, wherein determining augmented physiological data (408) further comprises:
- determining cleansed optical data (506) using the combined optical data and pressure data.

10. The physiological sensor system (200) of claim 8 or 9, wherein determining augmented physiological data (408) further comprises:
- determining an oscillometric measurement (508) of the blood pressure of the user using the combined optical data and pressure data.

11. The physiological sensor system (200) of claim 10, wherein the optical data includes an envelope of the optical data and determining an oscillometric measurement (508) includes analysing the envelope using the pressure data.

12. The physiological sensor system (200) of any of claims 1-11, further comprising a non-conducting dielectric layer (320) covering the RFF sensor (110).

13. The physiological sensor system (200) of claim 12, wherein the interface area is located on the non-conducting dielectric layer (320).

14. A method using a physiological sensor system (200) as defined in any of claims 1-13, wherein control circuitry (210) is configured to:
- determine (406) resonance data using the resonance radiofrequency sensor (110), optical data (404) using the optical sensor (120), load data (402) using the load sensor (130); the resonance data, the optical data and the load data being temporally synchronized,
- determine (408) augmented physiological data by combining the synchronized resonance data, optical data and load data.
